# EUROPEAN PATENT SPECIFICATION

(11) **EP 1 812 178 B1**
(45) Date of publication and mention of the grant of the patent: **04.07.2012**
(21) Application number: 05849305.7
(22) Date of filing: 18.11.2005
(51) Int. Cl.: B08B 9/00, A61L 33/00, B05D 1/18, B05D 3/02, B08B 9/04, B08B 9/043, A61B 19/00, B08B 1/00

(54) **Method for making of a cannula cleaning device**
Verfahren zur Herstellung einer Kanülenreinigungsvorrichtung
Methode de fabrication d'un dispositif pour le nettoyage des canules

(30) Priority: 18.11.2004 US 990503
(43) Date of publication of application: 01.08.2007
(73) Proprietor: The Ruhof Corporation, Mineola NY 11501 (US)
(72) Inventor: ESQUENET, Bernard, Old Brookville, New York 11545 (US); RUVINSKY, Lee, Mineola, New York 11501 (US); ESQUENET, Marc, Old Brookville, New York 11545 (US)
(74) Representative: Roberts, Gwilym Vaughan
(86) International application number: PCT/US2005/041831
(87) International publication number: WO 2006/055779

(56) References cited:
- US-A- 3 878 579
- US-A- 4 734 964
- US-A- 4 734 964
- US-A1- 2003 108 846
- US-A1- 2003 108 846
- US-A1- 2003 213 501
- US-A1- 2003 213 501

## Description

### Field of the Invention

The present invention relates to a method for forming a device, for cleaning of cannulas in medical instruments.

Medical procedures which use endoscopes, and other elongated instruments which are inserted through surgical openings, are relatively expensive products and must be used multiple times. Accordingly, such devices of necessity must be cleaned and sterilized repeatedly. Such instruments typically include long narrow cannulas through which surgical implements and other devices are passed in a surgical procedure. The lumens of such cannulas thus are subject to contamination by bodily fluids and materials and must be cleaned carefully before reuse. If they are not thoroughly cleaned prior to disinfection and sterilization, surgical debris can be passed to another patient leading to infection or other complications. Accordingly, it is very important to adequately clean the interiors of endoscopes and similar surgical instruments, which are often difficult to access.

Various techniques or devices have been previously proposed for cleaning the cannulas of the endoscopes, the simplest of which involves immersing the devices in solutions containing a detergent and/or an enzyme. Other applications use a small brush, constructed much like the conventional bottle brush having bristles locked between twisted wires, to reach the interior lumen of the cannula. Such brushes are not entirely effective as they do not carry the cleaning or enzymatic solution to the wall surfaces of the lumen. In addition, the bristles are liable to scratch or damage the interior surfaces of the endoscopes and leave hardened deposits thereon.

One solution to this problem was proposed in the United States Patent Application Publication No. US 2003/0213501A1 in which a hydrophilic polyurethane coating is deposited on the bristles of a conventional endoscopic cleaning brush. This coating is used to absorb an enzymatic cleaner and bring the cleaner to the interior surface of the lumen. However, it is believed that because the coating is still on the hard bristles of a conventional brush, some of the same problems that exist with conventional brushing solutions will remain with the device and method of the above-identified published application.
US 2003/0213 51 A1 discloses a scrubber for cleaning the lumen of a cannula of an endoscopic medical instrument, and a method of using said scrubber.
US 2003/0108846A1 discloses a disposable, handheld oral hygiene device comprising an elongated member and a swab attached to a longitudinal end of the elongated member.

### Objects of the Invention

An object of the present invention is to provide an improved cleaning device for the cannulas of endoscopes.

Another object of the invention is to provide a device for cleaning the lumen of cannulas which is relatively simple to manufacture and capable of carrying cleaning solution to all surfaces of the lumen.

Yet another object of the present invention is to provide a cleaning device for the lumen of cannulas and other medical equipment which is relatively inexpensive to manufacture and simple to use.

### Summary of the Present Invention

In a first aspect of the invention there is provided a method for making a cleaning device, the method as defined in claim I of the appended claims. In a second aspect there is provided a method of cleaning a cannula of a medical instrument, the method as defined in claim 4.

In accordance with an embodiment of the method of the present invention a device for cleaning the cannula of a medical instrument is provided which includes an elongated supported and guiding member have proximal and distal ends. The guiding member is preferably formed of a flexible material such as polypropylene.

A cleaning element is secured to the distal end of the support and guiding member. The cleaning element is formed of a substantially reticulated hydrophobic polyurethane foam having a plurality of pores formed within its structure. These pores define surfaces within the polyurethane foam. The surfaces of the foam are then coated with hydrophilic polyurethane foam. The hydrophilic polyurethane coated element is then immersed in an enzymatic cleaning solution so that the solution is absorbed throughout the cleaning element. The device may then be used to clean an endoscope by inserting the cleaning element in its lumen and manipulating it with the proximal end of the support rod.

In accordance with another embodiment of the method of the present invention the cleaning element may be dried after being immersed in the cleaning solution, so that the enzymatic materials remain in an essentially dried sponge. The sponge can be stored for later use. Thereafter, when it is necessary to clean an instrument the cleaning element is immersed in water and used as described above. It will be understood of course that in both cases the cleaning action is continued for a time sufficient to permit the solution to contact all surfaces of the lumen and clean or mechanically dislodge any debris therein.

The above, and other objects, features, and advantages of the present will be apparent to those skilled in the art from the following detailed description an illustrative embodiment of the invention when read in connection with the accompanying wherein:

### A Brief Description of the Drawings

Figure 1 is a plan view of a cleaning device constructed in accordance with the present invention.

Figure 2 is a sectional view taken along line 2-2 of Figure 1, showing the cleaning element in cross section in a slightly largely scale; and

Figure 3 is a schematic view on a much larger scale of a section of the cleaning element.

### A Detailed Description of the Invention

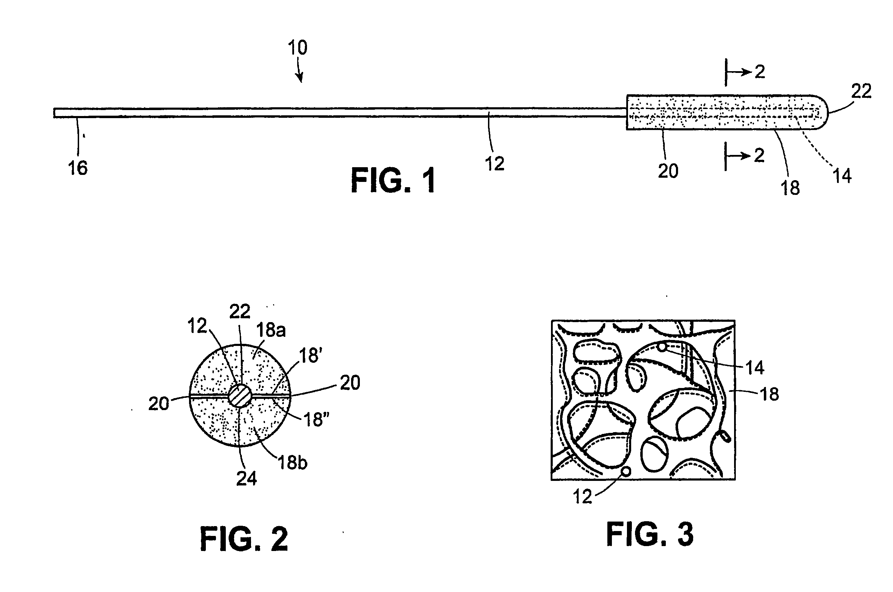

A cleaning device 10, constructed in accordance with the present invention, is illustrated in Figure 1 of the drawings. The device 10 includes a flexible elongated support and guiding rod 12 having a distal end 14 and a proximal end 16. Rod 12 is preferably formed of a very thin flexible material such as polypropylene.

A cleaning element 18 is secured to distal end 14, as described below. The cleaning element 18 is formed from a foam composite material such as is described in US Patent No. 6,617,014, the disclosure of which is incorporated herein by reference. This material is shown in a very enlarged scale in the schematic view of Figure 3. As seen therein the foam material forming the cleaning element 18 consists of a hydrophobic polyurethane foam which forms a reticulated scaffold 12. The hydrophobic scaffold 12 forms a backbone for an open cell foam coating 14 formed preferably of hydrophilic polyurethane. As described in patent No. 6,617,014 the hydrophobic polyurethane foam scaffold 12 is typically a reticulated foam made from water insoluble polyester, or polyester backbones and dilsocyanates as caps to the polyols. Other ingredients may be added to aid production. Such foams have a high porosity and are easy to fabricate. They further exhibit high tensile strength, elongation and tear properties to allow ease in cutting, shaping and provide long useful lives.

Hydrophilic polyurethanes are water-loving and absorb liquids to a greater degree than hydrophobic polyurethane. However, the physical strength and physical and tensile strength of hydrophilic materials is less than that of hydrophobic materials. Therefore, the composite material used as the cleaning element of the present invention provides benefits of both materials.

The cleaning element 18, as seen in Figure 2, is formed of two layers of the composite polyurethane material, 18a and 18b. These layers have opposing faces 18' and 18" when superimposed, again as seen in Figure 2. When they are placed with their opposing faces facing one another, about the distal end 14, layers 18a and 18b are die-cut and heat-sealed, using, for example, a U-shaped heat-sealing die with long leg portions, to seal the edges of layers 18a and 18b together along the seal line 20. The seal line will extend also along the tip or bite portion 22 of the layers across the end of the distal end 14 of rod 12.

Preferably, the opposing faces are 18' and 18" are adhered to the rod 12 about the surface 22 of the rod by a layer of adhesive material 24 applied to the rod before it is placed between the layers. Preferably, that adhesive material is an epoxy, as would be apparent to those skilled in the art. By adhering the cleaning element 18 to the distal end of the rod in this manner, the operator is assure that the cleaning element will not become dislodged within the lumen of the device being cleaned.

The cross section of the cleaning element 18 is selected to conform and closely match with the diameter lumen of the particular form of cannula to be cleaned. Preferably, its diameter or maximum dimension is slightly larger than that of the lumen so that the entire surface of the cleaning element engages and contacts the interior surface of the lumen of maximum cleaning function.

As described above, cleaning element 18 may be immersed in a cleaning solution and used immediately for cleaning a medical instrument. Preferably, the cleaning solution is an enzymatic cleaner of the type which can degrade, disperse, or dissolve biological contaminant. One preferred cleaner is sold by The Ruhof Corporation under the trademark ENDOZIME^{®}.

Once cleaning element 18 is secured to the distal end of the support rod 12, it preferably is immersed in the enzymatic cleaning solution and then passed through a dryer, of known construction, wherein excess water is removed without destruction of the enzymes. The thus dried device can be then be packaged or stored for a shipment to the end user.

When it is necessary to use the device, it can be removed from its package and immersed in water to reactivate the enzymatic cleaner. It is then inserted into the lumen of the medical device and moved vigorously within the lumen by manipulating the proximal end of rod 12 to release the enzymatic cleaner into contact with the entire inner surface of the cannula. The cleaning device is then removed and the cannula flushed with water or any suitable cleaning fluid. It thereafter may be sterilized if desired.

Although an illustrative embodiment of the invention has been described here in reference to accompanying drawings, it is to be understood that the invention is not limited to this precise embodiment and that various changes and modifications may be effected therein by those skilled in the art without the departing from the scope of this invention.

## Claims

1. A method for making a cleaning device (10) for cleaning the interior lumen of a cannula of a medical instrument comprising the steps of securing the distal end (14) of an elongated support and guiding rod (12) in a cleaning element (18); forming said cleaning element by providing a substantially reticulated hydrophobic polyurethane foam having a plurality of pores within the structure thereof defined by surface of the hydrophobic polyurethane foam and coating said surfaces of the hydrophobic polyurethane foam with hydrophilic polyurethane foam, immersing said cleaning element in an enzymatic cleaning solution **Characterized in that** it comprises the further step of drying said cleaning element after immersion in the enzymatic cleaning solution.

2. The method as defined in Claim I including the step of forming said cleaning element from two layers of said substantially reticulated polyurethane foam coated with said hydrophilic polyurethane foam and heat sealing said layers together about said distal end of said rod.

3. The method as defined in Claim 2 wherein said layers have opposing faces and said method includes the step of adhering said faces to said distal end of said rod.

4. A method of cleaning a cannula of a medical instrument comprising the steps of, forming a cleaning device according to the method of Claim 1, placing at least the cleaning element in water to wet said enzymatic cleaner, introducing the cleaning element into the cannula of a medical instrument and moving the cleaning element in the cannula of the instrument by manipulating it from the proximal end thereof.

## Patentansprüche

1. Verfahren zur Herstellung einer Reinigungsvorrichtung (10) zum Reinigen des inneren Hohlraums einer Kanüle eines medizinischen Instruments, enthaltend die Schritte des Befestigens des distalen Endes (14) einer länglichen Halterungs- und Führungsstange (12) in einem Reinigungselement (18), Bilden des Reinigungselements durch Vorsehen eines im Wesentlichen netzartigen hydrophoben Polyurethanschaums mit mehreren Poren innerhalb seiner Struktur, die durch die Oberfläche des hydrophoben Polyurethanschaums definiert sind, und Beschichten der Oberflächen des hydrophoben Polyurethanschaums mit hydrophilem Polyurethanschaum, Eintauchen des Reinigungselements in eine enzymatische Reinigungslösung, **dadurch gekennzeichnet, dass** es den weiteren Schritt des Trocknens des Reinigungselements nach dem Eintauchen in die enzymatische Reinigungslösung aufweist.

2. Verfahren nach Anspruch 1, enthaltend des Schritt des Bildens des Reinigungselements aus zwischen Schichten des im Wesentlichen netzartigen Polyurethanschaums, der mit dem hydrophilen Polyurethanschaum beschichtet ist, und Wärmeversiegeln der Schichten um das distale Ende der Stange.

3. Verfahren nach Anspruch 2, wobei die Schichten gegenüber liegende Flächen haben und das Verfahren den Schritt des Anklebens der Flächen an das distale Ende der Stange enthält.

4. Verfahren zum Reinigen einer Kanüle eines medizinischen Instruments, enthaltend die Schritte des Bildens einer Reinigungsvorrichtung nach dem Verfahren von Anspruch 1, Anordnen mindestens des Reinigungselements in Wasser, um den enzymatischen Reiniger zu benetzen, Einführen des Reinigungselements in die Kanüle eines medizinischen Instruments und Bewegen des Reinigungselements in der Kanüle des Instruments durch dessen Manipulieren von seinem proximalen Ende aus.

## Revendications

1. Procédé de fabrication d'un dispositif de nettoyage (10) pour nettoyer la lumière intérieure d'une canule d'un instrument médical, comprenant les étapes consistant à fixer l'extrémité distale (14) d'un support allongé et d'une tige de guidage (12) dans un élément de nettoyage (18) ; à former ledit élément de nettoyage en fournissant une mousse de polyuréthane hydrophobe sensiblement réticulée ayant une pluralité de pores au sein de sa structure définie par la surface de la mousse de polyuréthane hydrophobe et à revêtir lesdites surfaces de la mousse de polyuréthane hydrophobe d'une mousse de polyuréthane hydrophile en immergeant ledit élément de nettoyage dans une solution de nettoyage enzymatique, **caractérisé en ce qu'**il comprend l'étape supplémentaire consistant à sécher ledit élément de nettoyage après immersion dans la solution de nettoyage enzymatique.

2. Procédé selon la revendication 1, comprenant l'étape consistant à former ledit élément de nettoyage à partir de deux couches de ladite mousse de polyuréthane sensiblement réticulée revêtue de ladite mousse de polyuréthane hydrophile et à thermosceller lesdites couches l'une à l'autre autour de ladite extrémité distale de ladite tige.

3. Procédé selon la revendication 2, dans lequel lesdites couches ont des faces opposées et ledit procédé comprend l'étape consistant à faire adhérer lesdites faces à ladite extrémité distale de ladite tige.

4. Procédé de nettoyage d'une canule d'un instrument médical comprenant les étapes consistant à former un dispositif de nettoyage selon le procédé de la revendication 1, placer au moins l'élément de nettoyage dans l'eau pour mouiller ledit nettoyant enzymatique, introduire l'élément de nettoyage dans la canule d'un instrument médical et déplacer l'élément de nettoyage dans la canule de l'instrument en le manipulant à partir de son extrémité proximale.
